# EUROPÄISCHE PATENTSCHRIFT

(11) **EP 1 500 647 B1**
(45) Veröffentlichungstag und Bekanntmachung des Hinweises auf die Patenterteilung: **22.05.2013**
(21) Anmeldenummer: 04017388.2
(22) Anmeldetag: 22.07.2004
(51) Int. Cl.: C07D 201/12

(54) **Verfahren zur Behandlung Polyamid-haltiger Abfälle mit der Wiederaufbereitung des Depolymerisationsrückstandes**
Process for the treatment of polyamide containing waste by recycling of the depolymerization residue
Procédé de traitement de déchets contenant des polyamides par recyclage du résidu de dépolymérisation

(30) Priorität: 23.07.2003 DE 10333538
(43) Veröffentlichungstag der Anmeldung: 26.01.2005
(73) Patentinhaber: Lurgi Zimmer GmbH, 60295 Frankfurt am Main (DE)
(72) Erfinder: Kämpf, Rudolf, Dr., 63584 Haingründau (DE); Wolf, Reinhard, 63517 Rodenbach (DE); Seelig, Joachim, Dr., 63599 Biebergemünd (DE)
(74) Vertreter: Grünecker, Kinkeldey, Stockmair & Schwanhäusser

(56) Entgegenhaltungen:
- EP-A- 0 568 882
- EP-A- 0 633 246
- WO-A-00/64871
- WO-A-94/06763
- WO-A-97/03048
- GB-A- 862 567

## Beschreibung

Die vorliegende Erfindung betrifft ein kontinuierliches Verfahren zur Behandlung Polyamid-haltiger Abfälle unter Verbesserung der Wirtschaftlichkeit durch Rückführung von Wertstoffen aus der Spaltreaktorausschleusung bei der Wiederaufbereitung von Polyamid 6 durch säurekatalysierte Spaltung zu hochreinem und für die Polykondensation geeignetem Caprolactam aus Polyamidabfällen.

Eine Wiederaufbereitung durch Spaltung von sauberen, unkonfektionierten, fabrikneuen bei der Produktion direkt anfallenden Polyamidabfällen zu Produkten mit Qualitätsmerkmalen wie sie Neuware aufweist, ist ohne besondere Maßnahmen möglich. Wurden Gebrauchsartikel schon jahrelang benutzt oder sind Produkte aus einer Kunststoffsammlung zu verwenden, lässt sich dies nur mit erhöhtem technischen, energetischen und kostenmäßigem Aufwand durchführen. Es sind in diesem Falle, wie beispielsweise in DE 197 19 734, EP 0 681 896, US 5,598,980, DE 2416 573, DE 25 07 744 beschreiben, Reinigungs- und Sortiervorgänge notwendig um eine sortenreine Polyamid 6-Fraktion zu erhalten. Diese Verfahrensschritte sind unbedingt notwendig, da Kunststoffprodukte immer dem jeweiligen Einsatzgebiet angepasst werden. Aus diesem Grund enthalten sie hersteller- und herstellungsbedingt Zuschlagsstoffe, Farbmittel, Stabilisatoren, Glasfasern, etc., die eine Aufbereitung ohne zusätzliche Maßnahmen erschweren. Bei einer Wiederaufbereitung von Polyamidabfällen und der Rückführung in den Wertstoffkreislauf führt kein Weg an der Zerlegung in die Monomere vorbei, soll ein Produkt erzeugt werden, das sich nicht von Ware unterscheidet, die aus Monomeren der Syntheseroute hergestellt wurde.

Einer der wesentlichsten Verfahrenschritte, wie auch DE 887199, EP 0 875 505, DE 910056, US 4,605,672, JP 53-13636, der einschlägigen Literatur beispielweise Brandrup/Bittner/Michaeli/Menges "Die Wiederverwertung von Kunststoffen", S. 513-520 Verlag Carl Hanser München Wien 1995 zu entnehmen ist, stellt die alkali- oder säurekatalysierte Spaltung und die Abtreibung von Caprolactam mittels Wasserdampfdestillation dar. Da Polyamid 6 Produkte wie oben erwähnt, meist nicht das einzige Material ist, das in einen Spaltreaktor eingebracht wird, müssen Stör- oder Zuschlagsstoffe kontinuierlich oder in Zeitintervallen ausgeschleust werden. Die Stör- und Zuschlagsstoffe bei einer Wiederaufbereitung von Polyamid 6 -Teppichen entstehen dadurch, dass bei der Teppichfabrikation auf ein Trägergewebe, z.B. Polypropylenvlies, die als Flor oder auch Nutzschicht bezeichneten Fasern genadelt werden. Nach der Aufbringung des Flors erfolgt eine Fixierung mittels einer Klebeschicht, auf die dann ein mit Füllstoffen, wie Kreide, gefüllter Polymerschaumrücken aufgetragen wird. Bei einer Wiederaufbereitung wird aus zerkleinertem Teppichmaterial der Flor bzw. die Nutzschicht separiert und einer Depolymerisation zur Rückgewinnung des Monomeren zugeführt. In der Depolymerisation erfolgt beispielsweise nach den aus DE 887199, EP 0 875 504, DE 910056, US 4,605,762, JP 53-13636 bekannten Verfahren mittels Phosphorsäure eine Spaltung der Polymerketten und eingeleiteter Dampf treibt das entstandene Caprolactam als Wasserdampfgemisch ab.

Die mechanische Aufbereitung und Trennung nach der Schwimm-Sink-Zentrifugen-Technologie, wie sie beispielsweise in Teppichaufbereitungsanlagen in USA oder Deutschland ausgeführt ist, separiert nicht hundertprozentig reinen Flor oder Nutzschicht aus Polyamid 6. Es verbleiben noch Störstoffe, wie Anteile an SBR-Schaumrücken, Kreide aus der Aufbereitung, Russ, Geweberückstände sowie andere Füllstoffe. Im Laufe des kommerziellen Betriebs einer Teppichaufbereitung hat es sich als vorteilhaft erwiesen, in dem Kemstück, nämlich der Caprolactam-Rückgewinnungsanlage diese Nebenbestandteile kontinuierlich, durch Analysen und online Messungen des Phosphorsäuregehaltes gesteuert, als Rückstand auszuschleusen. Besonders vorteilhaft hat sich das Überwachen und Steuern der auszuschleusenden Masse durch Analysen und online Messungen des Phosphorsäuregehaltes in Strom im Hinblick auf den wirtschaftlichen Betrieb einer solchen Anlage erwiesen, da dadurch zum einen der Verbrauch an Phosphorsäure und zum anderen der Verbrauch an Alkali zur Neutralisation des Rückstandes minimiert werden konnte. Ferner konnten durch Messung und Überwachung des Stickstoffgehaltes im auszuschleusenden Stoffstrom vorteilhaft die Polymerkettenspaltung und somit die Ausbeute an Caprolactam gegenüber einer nicht Stickstoff überwachten Betriebsweise gesteigert werden.

Nachteilig an der vorstehend beschriebenen Verfahrensweise ist die Tatsache, dass der ausgeschleuste Rückstand mit erheblichen Mengen an Alkali neutralisiert wird, um die mit ausgeschleuste und nicht verbrauchte Phosphorsäure zu binden.

Die WO 94/06763, WO 00/64871 und EP-A-0 633 246 beschreiben Verfahren zur Behandlung von Polyamid-haltigen Abfällen.

Daher liegt der vorliegenden Erfindung die Aufgabe zugrunde, ein wirtschaftlicheres Verfahren zur Aufbereitung des ausgeschleusten Rückstandes zur Verfügung zu stellen.

Die Lösung der Aufgabe ist ein Verfahren zur Behandlung Polyamid-haltiger Abfälle gemäß Anspruch 1. Bevorzugte Ausführungsformen werden in den Unteransprüchen 2 bis 8 beschrieben.

Die im erfindungsgemäßen Verfahren verwendbaren Polyamid-haltigen Abfälle sind vorzugsweise ausgewählt aus gebrauchten, verschmutzten oder dem Recyclingkreislauf entnommenen Polyamid 6 Abfällen. Die Polyamid-haltigen Abfälle sind insbesondere bevorzugt ausgewählt aus der Gruppe, bestehend aus Polyamid-haltigen Formkörpern, wie Teilen für Fahrzeuge, Polyamid-haltigen Spritzgussteilen mit Glasfasern und anderen Zuschlägen sowie Polyamid-haltigen Fasern, Teppichen, Teppichbodenbelägen und anderen Polyamid-haltigen Gegenständen, wie Kleidung. Das Polyamid-haltige Material, das der Depolymerisation zugeführt wird, kann außerdem Nichtpolymere und artfremde Zuschlagsstoffe enthalten.

Die Polyamid-haltigen Abfälle können bevorzugt vor dem Durchführen der Depolymerisation in Stufe a) aufgeschmolzen werden. Außerdem können diesen weitere Produkte zugeführt werden, die aus einer Caprolactamdestillation stammen.

Die Depolymerisation in Stufe a) des erfindungsgemäßen Verfahrens wird nach bekannten Verfahren durchgeführt, wie einer sauren Spaltung, beispielsweise mittels Phosphorsäure, einer alkalischen Spaltung oder einer Dampfspaltung. Vorzugsweise wird die Depolymerisation in Stufe a) mittels Phosphorsäure durchgeführt. Das nach der Depolymerisation erhaltene Caprolactam-Rohmaterial kann sodann aufkonzentriert und der weiteren gewünschten Verwendung zugeführt werden, wie der Herstellung von Polyamid. Der weitere nach der Depolymerisation erhaltene Strom umfasst die Rückstände der jeweiligen Spaltungen, nämlich unter anderem Nebenbestandteile und Zuschlagsstoffe. Die im Strom (3) enthaltenen Nebenbestandteile sind wertvolle Bestandteile, die weiter verwendet werden können, wie Spaltsäuren oder-alkali, Spaltprodukte der Polyamidspaltung und 6-Aminocapronsäure, Caprolactam und cyclische und lineare Polyamid-oligomere. Wenn eine saure Spaltung mittels Phosphorsäure durchgeführt wird, umfassen die Nebenbestandteile Phosphorsäure, Caprolactam, Spaltprodukte der Polyamidspaltung, cyclische und lineare Polyamid-oligomere und 6-Aminocapronsäure.

Die in Strom (3) außerdem enthaltenen Zuschlagstoffe sind nicht weiter verwendbar und umfassen beispielsweise Glasfasern, Kreide, Baryt, Russ, Kaolin, Bentonit, Silikate und Farbstoffe.

Der Strom (3) wird sodann in Stufe b) des erfindungsgemäßen Verfahrens ausgelaugt. "Auslaugen" im Sinne der vorliegenden Erfindung bedeutet das In-Kontakt-bringen des Strom (3) mit einem Extraktionsmittel.

Das Auslaugen erfolgt bevorzugt mit einem einzelnen oder einer Mischung mehrerer Extraktionsmittel ausgewählt aus der Gruppe, bestehend aus Wasser, Alkoholen, Alkylcarbonaten, Aminen und deren wässrigen Gemischen. Insbesondere bevorzugt wird reines Wasser verwendet. Es können aber auch beispielsweise 10-80 Massen%-ige wässrige Mischungen aus Methanol, Ethanol, Isopropanol oder Ethylencarbonat vorteilhaft eingesetzt werden. Die niedrigen Alkohole selbst sind dagegen wegen ihrer niedrigen Siedepunkte und der leichten Entflammbarkeit ihrer Dämpfe weniger geeignet.

Zur Erreichung der notwendigen Kontaktzeit zwischen dem Strom (3), der Feststoffe, nämlich Nebenbestandteile und Zuschlagstoffe, enthält oder aus solchen Feststoffen besteht, und dem Extraktionsmittel, wird vorteilhafterweise eine Aufschlämmung aus dem feststoffhaltigen Strom (3) und dem Extraktionsmittel hergestellt.

Wenn das erfindungsgemäße Verfahren in Stufe a) mittels Phosphorsäure durchgeführt wird, enthält der Strom (3) unter anderem 6-Aminocapronsäure, Caprolactam und Phosphorsäure, also drei wertvolle Rohstoffe. Beim Durchführen der Stufe b) des erfindungsgemäßen Verfahrens löst sich Aminocapronsäure, ebenso wie das Spaltprodukt Caprolactam und die Spaltsäure, wie Phosphorsäure, sehr gut in dem Extraktionsmittel, wie Wasser, Alkoholen, Alkylcarbonaten oder deren Gemischen, wobei jedoch die Löslichkeit in Wasser höher ist, als in Gemischen aus Alkoholen oder Aminen. Daher ist es bevorzugt, Wasser als Extraktionsmittel zu verwenden.

So lösen sich beispielsweise in Wasser 20°C 800 g/l Aminocapronsäure und 4560 g/l Caprolactam. Die gute Löslichkeit führt dazu, dass sich Lösungen leicht übersättigen lassen und zur Extraktion bei höheren Temperaturen nur geringe Mengen an Wasser notwendig sind.

Es können vorteilhafterweise etwa 150 bis etwa 5000 g Extraktionsmittel, insbesondere Wasser, pro 100 g Strom (3) in Stufe b) verwendet werden.

Es ist bevorzugt die Stufe b) bei erhöhten Temperaturen durchzuführen, insbesondere von Raumtemperatur bis etwa 5 bis etwa 10 °C unterhalb des Siedepunkts des Extraktionsmittels, bevorzugter von etwa 30 °C bis etwa 5 °C unterhalb des Siedepunkts des Extraktionsmittels. Falls Wasser als Extraktionsmittel verwendet wird, wird Stufe b) bevorzugt bei etwa 40°C bis etwa 95°C durchgeführt. Werden Extraktionsmittel, wie niedere Alkohole, Amine oder Alkylcarbonate eingesetzt, so liegen deren Siedepunkte meist unterhalb dessen von Wassers. Um einen gewissen Abstand zum Siedepunkt zu halten, sollte die höchste Temperatur bei der Extraktion mindestens 5 bis 10°C unterhalb des Siedepunktes liegen. Vorteilhaft werden cyclische Carbonate als Extraktionsmittel verwendet, da sie höhere Siedepunkte als Wasser und ein ähnlich hohes Löse- und Übersättigungsverhalten wie Wasser haben und daher bis zu Temperaturen von 220° C angewendet werden können.

Des weiteren ist es bevorzugt, dass der Strom (3) Feststoffe mit einer Korngröße von bis zu etwa 5 mm insbesondere bis zu etwa 2 mm umfasst.

Bevorzugt wird Stufe b) des erfindungsgemäßen Verfahrens etwa 5 Minuten bis etwa 4 Stunden durchgeführt.

Nach dem Auslaugen in Stufe b) des Verfahrens wird somit ein Gemisch erhalten, in dem Feststoff, der Zuschlagsstoff und gegebenenfalls weitere Nebenbestandteile enthält, und Extraktionsmittel mit darin gelösten Nebenbestandteilen, wie 6-Aminocapronsäure, Phosphorsäure und anderen wertvollen Polyamidspaltprodukten, enthalten sind. Dieses Gemisch ist insbesondere eine Aufschlämmung. Vorzugsweise wird sodann dieses Gemisch in einen flüssigen und einen feststoffhaltigen Teil getrennt. Das erfindungsgemäße Verfahren umfasst daher eine weitere Stufe c), nämlich Abtrennen des Feststoffs vom Extraktionsmittel mit darin gelösten Nebenbestandteilen.

Das Trennen des nach Stufe b) enthaltenen Gemisches, insbesondere einer Aufschlämmung, in Stufe c) des Verfahrens kann bevorzugt durch mechanisches Trennen, wie durch Ruhenlassen des Gemisches, insbesondere der Aufschlämmung, wodurch die Feststoffe absinken, und Abtrennen des flüssigen Überstands oder durch Zentrifugieren erfolgen. Bevorzugt wird Stufe c) in einem Feststoffabscheider durchgeführt.

Nach Stufe c) wird der abgetrennte flüssige Teil, nämlich das Extraktionsmittel mit darin gelösten Nebenbestandteilen, wieder in Stufe a) zurückgeführt.

Außerdem wird nach Stufe c) erhaltene feststoffhaltige Teil wiederum in Stufe b) des Verfahrens zurückgeführt, d.h. einem weiteren Auslaugen des Feststoffs mit Extraktionsmittel zugeführt, wobei dann dieser Feststoff dem Strom (3) beim erstmaligen Durchführen des Verfahrens entspricht. Der nach dem Durchführen der Stufe c) erhaltene feststoffhaltige Teil kann auch gemeinsam mit dem Strom (3) in Stufe b) des erfindungsgemäßen Verfahrens ausgelaugt werden. Auch nach dem zweiten Durchführen der Stufe b) wird ein Extraktionsmittel mit darin gelösten Nebenbestandteilen und ein feststoffhaltiger Teil erhalten, wobei dieses Gemisch wiederum bevorzugt in einen flüssigen und einen feststoffhaltigen Teil getrennt wird. Der flüssige Teil mit darin enthaltenen Nebenbestandteilen kann dann wieder Stufe a) zugeführt werden.

Wie dem Vorstehenden zu entnehmen können die Stufen b) und c) des erfindungsgemäßen Verfahrens somit mehrmals durchgeführt werden, bevorzugt ein bis etwa 5-mal, wobei jeweils der nach Stufe c) erhaltene flüssige Teil mit wertvollen Nebenbestandteilen in Stufe a) zurückgeführt werden kann und der nach Stufe c) erhaltene feststoffhaltige Teil wieder in Stufe b) zurückgeführt wird.

Der nach dem Durchführen des Stufe c) erhaltene flüssige Teil, der Extraktionsmittel und Nebenbestandteile enthält hat vorzugsweise eine Konzentration von bis zu 90 % der Sättigungsgrenze der jeweiligen Bestandteile in dem verwendeten Extraktionsmittel.

Der nach dem Durchführen der Stufe c) erhaltene feststoffhaltige Teil kann, wenn dessen Gehalt an wertvollen Nebenbestandteilen niedrig ist, nach dem Auslaugen aus dem Kreislauf ausgeschleust werden.

Das erfindungsgemäße Verfahren ermöglicht eine bis zu 80%-ige Rückgewinnung der restlichen Nebenbestandteile der Depolymerisation, nämlich der Wertstoffe im ausgeschleusten Rückstand. Insbesondere können durch das Durchführen des erfindungsgemäßen Verfahrens mehr als 80 Massen % an Aminocapronsäure , mehr als 70% andere Polyamidspaltprodukte, wie beispielsweise Caprolactam und nicht umgesetzte Phosphorsäure aus dem Strom (3) herausgelöst werden.

Durch das erfindungsgemäße Verfahren wird eine Verbesserung der Wirtschaftlichkeit einer Polyamid-Depolymerisationsanlage erreicht, indem der ausgeschleuste Rückstand aus der Depolymerisation durch mindestens einmaliges Auslaugen von löslichen Bestandteilen befreit wird und dadurch dessen wertvollen Bestandteile für eine weitere Verwendung zur Verfügung stehen. Die Aufbereitung des ausgeschleusten Rückstandes aus dem Depolymerisationsreaktor hat sich somit vorteilhaft auf die Wirtschaftlichkeit der Wiederaufbereitung ausgewirkt, und ist dadurch gekennzeichnet, dass durch extraktives Auslaugen des Rückstandes Wertstoffe wie Phosphorsäure, 6-Aminocapronsäure und andere noch lösliche Wertstoffe zurückgewonnen werden. Eine Rückführung eines Extraktes vom Rückstand aus dem Depolymerisationsreaktor mit der darin im wesentlichen enthaltenen der Phosphorsäure und 6-Aminocapronsäure senkte zum einen vorteilhaft den Säureverbrauch um 3 bis 5% und erhöhte zum anderen überraschenderweise den Gehalt an Caprolactam in den Brüden, also dem Caprolactam-Rohmaterial, aus dem Depolymerisationsreaktor. Die Rückführung von 6-Aminocapronsäure an den Ort des Reaktionsgeschehens bzw. ihrer Entstehung hat sich überraschenderweise als besonders vorteilhaft erwiesen, da bei einer Nachprüfung durch gezielte Einspeisung von nur 6-Aminocapronsäure in Konzentration und der Menge der Ausschleusung entsprechend, diese Maßnahme zu einer Steigerung der Ausbeute an Caprolactam in den abgestrippten Brüden des Depolymerisationsreaktor um mindestens 3 bis 5 Massen % geführt hat. Diese Zunahme steht vermutlich in einem thermodynamischen/reaktionstechnischen Gleichgewicht mit Caprolactam und wird als Folge der Hydrolyse aus dem ringförmigen Lactam durch Einbau eines Wassermoleküls gebildet.

Wertstoffe wie Phosphorsäure, 6-Aminocapronsäure und andere noch lösliche Wertstoffe werden zurückgewonnen und als Extrakt vom Rückstand aus dem Depolymerisationsreaktor in den Depolymerisationsreaktor zurückgeführt. Die Rückspeisung von 6-Aminocapronsäure in den Depolymerisationsreaktor führt zu einer Steigerung der Caprolactamausbeute.

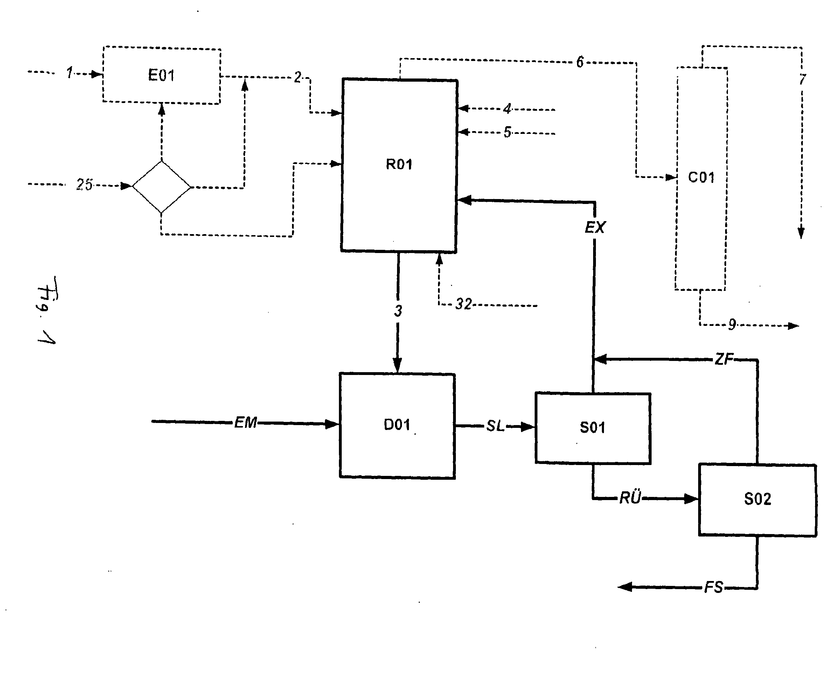

Figur 1 zeigt eine Anlage, die zum Durchführen des erfindungsgemäßen Verfahrens geeignet ist und es bedeuten

### Ausrüstung:

- E01: Aufschmelzeinrichtung
- C01: Aufkonzentrierungseinrichtung
- D01: Extraktionsapparat
- R01: Depolymerisationsreaktor
- S01: erster Feststoffabscheider
- S02: zweiter Feststoffabscheider

### Stoffströme:

- 1: Polyamid 6 Abfall
- 2: Schmelze
- 3: Ausschleusung Depolymerisation
- 4: Dampf Polyamid Extraktion/Konzentration
- 5: Phosphorsäure
- 6: Rohlactam/Wasser
- 7: Dampf
- 8: Rohlactam Konzentrat
- 25: Rohcaprolactam Rückstand
- 32: Polyamid-Extrakt-Konzentrat aus der Produktgranulatextraktion
- EM: Extraktionsmittel
- EX: Extrakt
- FS: Feststoffausschleusung
- RÜ: Separationsrückstand
- SL: Suspension Rückstand Extraktionsmittel
- ZF: Zentrifugat

Das erfindungsgemäße Verfahren zur Steigerung der Wirtschaftlichkeit der Behandlung von Polyamid-Abfällen wird nunmehr anhand von Figur 1 näher erläutert.

Durch mechanische Verfahren zurückgewonnen Polyamid 6 Abfälle werden, wie Figur 1 zeigt, über die Zuführung 1 einem Aufschmelzextruder E01 mit anderen aus einer Caprolactamdestillation stammenden Produkten zugeführt. Die den Aufschmelzapparat E01 verlassende Polymerschmelze wird über die Zuleitung 2 dem Depolymerisationsreaktor R01 kontinuierlich zugeführt, in dem nach den bekannten Verfahren nach DE 887199 EP 0 875 504, DE 910056, US 4,605,762 und JP 53-13636 mittels Phosphorsäure 5 eine Spaltung der Polymerketten erfolgt. Dabei wird Caprolactam gebildet, das durch eingeleiteten Dampf 4 über Kopf 6 abgetrieben und einer Aufkonzentrierungseinrichtung C01 zugeführt wird.

Da die mechanische Aufbereitung und Trennung nach der Schwimm-Sink-Zentrifugen-Technologie nicht von Störstoffen vollständig befreites Polymer dem Extruder E01 anliefert, in dem beispielsweise noch Anteile an SBR-Schaumrücken und Kreide aus der Aufbereitung von Teppichen, Russ, Geweberückstände sowie andere Füllstoffe im Stoffstrom 2 enthalten sind, bleibt ein Rückstand im Depolymerisationsreaktor R01 zurück, der kontinuierlich oder in Zeitintervallen ausgeschleust werden muss. Obwohl es sich beim kommerziellen Betrieb einer Caprolactam-Rückgewinnungsanlage als günstig erwiesen hat, diese Nebenbestandteile kontinuierlich und durch online Analysen und Messungen des Phosphorsäuregehaltes gesteuert über die Rückstandsleitung 3 auszuschleusen, konnte diese Maßnahme nicht verhindern, dass Verluste an Wertstoffen auftreten. Zusätzliche Kosten entstehen durch die Zugabe von Lauge, um den säurehaltigen Rückstand 3 entsorgungsfähig zu machen.

Es hat sich für den Betrieb Depolymerisationsanlage als Verbesserung der Wirtschaftlichkeit erwiesen, durch ein Auslaugen des Feststoffes in einem Extraktionsapparat D01 wertvolle, nicht umgesetzte Phosphorsäure und das bei der Polyamidspaltung als Nebenprodukt auftretende 6-Aminocapronsäure zurück zu gewinnen. Die den Extraktionsapparat D01 verlassende Aufschlämmung SL wird einem Feststoffabscheider S01 zugeführt und die über dem Rückstand stehende Flüssigkeit EX in den Depolymerisationsreaktor zurückgeführt. Der vom Boden des ersten Abscheider S01 kommende Feststoff RÜ gelangt in die zweite Feststoffabscheidung S02, in der beispielsweise durch Zentrifugen, eine weitestgehende Entfernung der Flüssiganteile erfolgt. Auch dieser als Zentrifugat ZF bezeichnete Flüssigkeitsstoffstrom geht vereinigt mit dem Extrakt EX aus Stufe 1 zurück in den Depolymerisationsreaktor R01. Der ausgelaugte und nahezu trockene Feststoff FS kann zusammen mit Klärschlamm entsorgt werden.

Der Erfindung wird nachstehend anhand eines Beispiels näher erläutert:

### Beispiel

Aus einem Spaltkessel einer Teppichrecyclinganlage zur Wiedergewinnung von Caprolactam aus sortierten Nylon 6 Bodenbelägen wird über ein Bodenauslassventil und eine Austragspumpe kontinuierlich ein Rückstandsstrom abgezogen. Dieser Strom wird so bemessen, dass die Rückstandskonzentration im Spaltkessel konstant bleibt. Der Mengenstrom bewegt im Bereich einer dem Fachmann bekannten Abschlämmung aus Eindampfanlagen und liegt bei 3 bis 5 Massen% des zugeführten Stromes 2. Der Rückstandsstrom 3 setzt sich zum Hauptteil aus Mischsalzen von Alkali- Erdalkalicarbonaten, - phosphaten, nicht umgesetzten Polyamiden und Aminocapronsäure zusammen. Aminocapronsäure steht als hydratisierte Form des cyclischen Caprolactam im Gleichgewicht mit diesem und wird zur Erhaltung des Gleichgewichtes immer wieder nachgebildet, so es aus dem Prozess entfernt wird.

Die aus dem Spaltreaktor R01 ausgetragene Schmelze 3 weist je nach Betriebsweise des Kessels eine Temperatur zwischen von 150°C bis 300°C auf und enthält je nach Betriebsweise noch 3 bis 12 Massen% Aminocapronsäure und andere Polyamidspalt-produkte. Der schmelzflüssige Reaktoraustrag 3 wird dem Extraktionsapparat D01 zugeführt, in dem während der Abschreckphase schon aus dem Produkt lösliche Anteile herausgelöst werden.

Durch das Einbringen der heißen Schmelze in das Extraktionsmittel, beispielsweise in Wasser, ergibt sich neben der feinen Verteilung auch noch die Aufheizung des Wassers oder Extraktionsmittels, die durch geeignete Zufuhr von frischem oder recycliertem Wasser bzw. Extraktionsmittel EM bis zum Sieden gesteigert werden kann. Es ist vorteilhaft mit Wasser bei Temperaturen um 90°C zu arbeiten, da hier ein betriebliches wirtschaftliches Optimum zwischen verdampfender Wassermenge und Sättigungskonzentration an Aminocaprosäure vorliegt. Durch eine Umwälzvorrichtung wird der fein verteilte Feststoff mit einer mittleren Verweilzeit von 5 Minuten bis 4 Stunden, vorteilhaft 1,5 Stunden, mit dem Extraktionsmittel in Verbindung gebracht, wobei sich mehr als 80 Massen % an Aminocapronsäure, Polyamidspaltprodukte und nicht umgesetzte Spaltsäure, beispielweise Phosphorsäure aus der anorganischen Matrix lösen. Die feststoffhaltige Lösung wird mittels Pumpen zur Trennung des Feststoffes von der Lösung in die folgenden Abscheidestufen geschickt. Im ersten Feststoffabscheider S01, der aus einem konischen Sedimentationsbehälter besteht, können sich die Feststoffpartikel mit einer mittleren Verweilzeit von 5 Minuten bis 12 Stunden bei Temperaturen zwischen 15°C und 90°C absetzen. Die überstehende zwischen 5 und 75 Massen% Aminocapronsäure, Polyamidspaltprodukte und Spaltsäure enthaltende Lösung EX wird mittels Pumpen zum Spaltkessel R01 zurückgeführt.

Die am Boden lagernden festen Bestandteile RÜ werden von dort mittels einer Schneckenfördervorrichtung in den zweiten Feststoffabscheider S02 gefördert um die anhaftende Lösung zurückzugewinnen. Dieser zweite Feststoffabscheider besteht aus einem kontinuierlich betriebenen Dekanter, mit dessen Hilfe dem Feststoff anhaftende Lösung fast vollständig entzogen wird.

Der Dekanter weist ausserdem noch eine Nachspüleinrichtung auf, mit deren Hilfe der Feststoff mit reinem Extraktionsmittel nachgewaschen werden kann.

Der den Dekanter verlassende Feststoffstrom FS weist nur noch geringe Feuchte von unter 10 Massen% auf und eignet sich ohne weitere Trocknung beispielsweise wegen seines hohen Erdalkali- und Phosphorgehaltes als Dünger.

Die abgetrennte Flüssigkeit ZF wird mit dem Extraktstrom EX aus der ersten Feststoff-abscheidung vereinigt und gemeinsam dem Spaltkessel R01 zugeführt.

## Patentansprüche

1. Verfahren zur Behandlung Polyamid-haltiger Abfälle, umfassend
a) Depolymerisieren der Polyamid-haltigen Abfälle, wodurch ein Caprolactam-Rohmaterial (6) und ein Strom (3) erhalten wird, der Nebenbestandteile und Zuschlagsstoffe der Depolymerisation umfasst,
b) wenigstens einmal Auslaugen des Stroms (3) mittels eines Extraktionsmittels, und
c) wenigstens einmaliges Trennen eines nach Stufe b) erhaltenen Gemisches in einen flüssigen Teil und einen feststoffhaltigen Teil,
**dadurch gekennzeichnet, dass** der erhaltene feststoffhaltige Teil aus Stufe c) in Stufe b) entweder vollständig oder als Teilstrom des Verfahrens zurückgeführt wird, wobei der nach Stufe c) erhaltene flüssige Teil in Stufe a) zurückgeführt wird.

2. Verfahren nach Anspruch 1, wobei die Polyamid-haltigen Abfälle ausgewählt sind aus der Gruppe, bestehend aus Polyamid-haltigen Formkörpern, Polyamid-haltigen Spritzgussteilen mit Glasfasern und anderen Zuschlägen sowie Polyamid-haltigen Fasern, Teppichen, Teppichbodenbelägen und anderen Polyamid-haltigen Gegenständen des täglichen Lebens.

3. Verfahren nach einem der vorstehenden Ansprüche, wobei die Depolymerisation in Stufe a) durch saure, alkalische und/oder Dampf-Spaltung durchgeführt wird.

4. Verfahren nach Anspruch 3, wobei die saure Spaltung mittels Phosphorsäure durchgeführt wird.

5. Verfahren nach Anspruch 4, wobei der Strom (3) als Nebenbestandteile Phosphorsäure, Caprolactam andere Polyamidspaltprodukte und 6-Aminocapronsäure umfasst.

6. Verfahren nach einem der vorstehenden Ansprüche, wobei das Auslaugen in Stufe b) mit einem einzelnen oder mehreren Extraktionsmitteln, ausgewählt aus der Gruppe, bestehend aus Wasser, Alkoholen, Alkylcarbonaten, Aminen und deren wässrigen Gemischen durchgeführt wird.

7. Verfahren nach einem der vorstehenden Ansprüche, wobei die Temperatur des Extraktionsmittels in Stufe b) von 30°C bis 5°C, bevorzugt von 10°C bis 5°C, unterhalb des Siedepunkts des Extraktionsmittels liegt.

8. Verfahren nach einem der vorstehenden Ansprüche, wobei Stufe c) in einem Feststoffabscheider durchgeführt wird.

## Claims

1. Method for the treatment of waste containing polyamide, comprising:
a) depolymerization of the waste containing polyamide, whereby a caprolactam raw material (6) and a flow (3), comprising secondary constituents and additives from the depolymerization, are obtained, and
b) leaching, at least once, the flow (3) with an extracting agent, and
c) separation, at least once, of a mixture obtained after stage (b) into a liquid part and a part containing solids,
**characterized in that** the obtained part containing solids of stage (c) in stage (b) is either returned completely or as a partial flow of the process, wherein the liquid part obtained after stage (c) is returned to stage (a).

2. Method according to claim 1, wherein the waste containing polyamide is selected from the group consisting of molded parts containing polyamide, injection molded parts with glass fibers containing polyamide and other additives and fibers, carpets, carpet floor coverings containing polyamide, and other objects containing polyamide from daily life.

3. Method according to any of the preceding claims, wherein the depolymerization is carried out in stage (a) through acidic, alkaline, and/or vapour cracking.

4. Method according to claim 3, wherein the acidic cracking is carried out using phosphoric acid.

5. Method according to claim 4, wherein the flow (3) comprises as secondary constituents phosphoric acid, caprolactam, other polyamide cracking products, and 6-aminocaproic acid.

6. Method according to any of the preceding claims, wherein the leaching in step (b) is carried out with a single or with more extraction agents, selected from the group consisting of water, alcohols, alkyl carbonates, amines, and their aqueous mixtures.

7. Method according to any of the preceding claims, wherein the temperature of the extraction agent in stage (b) is 30°C to 5°C, preferred from 10°C to 5°C, less than the boiling point of the extraction agent.

8. Method according to any of the preceding claims, wherein stage (c) is carried out in a solid separator.

## Revendications

1. Procédé de traitement de déchets contenant des polyamides, comprenant
a) la dépolymérisation des déchets contenant des polyamides, par laquelle est obtenu un matériau brut de caprolactame (6) et un courant (3) contenant des composants secondaires et des additifs de la dépolymérisation,
b) au moins une passe de lixiviation du courant (3) à l'aide d'un agent d'extraction, et
c) au moins une passe de séparation d'un mélange obtenu à l'étape b) en une fraction liquide et une fraction contenant des solides,
**caractérisé en ce que** la fraction contenant des solides obtenue à l'étape c) est renvoyée à l'étape b) soit entièrement, soit comme courant partiel du procédé, dans lequel la fraction liquide obtenue à l'étape c) est renvoyée à l'étape a).

2. Procédé selon la revendication 1, dans lequel les déchets contenant des polyamides sont choisis parmi le groupe constitué par des éléments moulés contenant des polyamides, des éléments moulés par injection contenant des polyamides, avec des fibres de verre et d'autres charges ainsi que des fibres contenant des polyamides, des tapis, des tapis de revêtements de sol, et d'autres produits manufacturés d'usage courant contenant des polyamides.

3. Procédé selon l'une des revendications précédentes, dans lequel la dépolymérisation de l'étape a) est effectuée par un clivage acide, un clivage basique et/ou un craquage à la vapeur.

4. Procédé selon la revendication 3, dans lequel le clivage acide est effectué à l'acide phosphorique.

5. Procédé selon la revendication 4, dans lequel le courant (3) comprend comme composants secondaires de l'acide phosphorique, du caprolactame, d'autres produits de décomposition du polyamide et de l'acide 6-aminocaproïque.

6. Procédé selon l'une des revendications précédentes, dans lequel la lixiviation de l'étape b) est réalisée avec un ou plusieurs agents d'extraction, sélectionnés parmi le groupe constitué par l'eau, les alcools, les carbonates d'alkyle, les amines et les mélanges aqueux de ceux-ci.

7. Procédé selon l'une des revendications précédentes, dans lequel la température de l'agent d'extraction à l'étape b) est comprise entre 30°C et 5°C, de préférence entre 10°C et 5°C, sous le point d'ébullition de l'agent d'extraction.

8. Procédé selon l'une des revendications précédentes, dans lequel l'étape c) est réalisée dans un séparateur de solides.
